# EUROPÄISCHE PATENTSCHRIFT

(11) **EP 2 694 468 B1**
(45) Veröffentlichungstag und Bekanntmachung des Hinweises auf die Patenterteilung: **18.01.2017**
(21) Anmeldenummer: 12711135.9
(22) Anmeldetag: 20.03.2012
(51) Int. Cl.: C07C 227/26, C07C 229/16

(54) **VERFAHREN ZUR HERSTELLUNG VON WÄSSRIGEN LÖSUNGEN VON METHYLGLYCIN-N,N-DIESSIGSÄURE-TRIALKALIMETALLSALZEN**
METHOD FOR PRODUCING AQUEOUS SOLUTIONS OF METHYLGLYCINE-N,N-DIACETIC ACID TRIALKALI METAL SALTS
PROCÉDÉ DE PRÉPARATION DE SOLUTIONS AQUEUSES DE SELS MÉTALLIQUES ALCALINS D'ACIDE N,N-DIACÉTIQUE DE MÉTHYLGLYCINE

(30) Priorität: 04.04.2011 EP 11161045; 07.02.2012 EP 12154201
(43) Veröffentlichungstag der Anmeldung: 12.02.2014
(73) Patentinhaber: BASF SE, 67056 Ludwigshafen am Rhein (DE)
(72) Erfinder: OFTRING, Alfred, 67098 Bad Dürkheim (DE); BRAUN, Gerold, 67071 Ludwigshafen (DE); LAUTERBACH, Arnulf, 67067 Ludwigshafen (DE)
(74) Vertreter: BASF IP Association
(86) Internationale Anmeldenummer: PCT/EP2012/054889
(87) Internationale Veröffentlichungsnummer: WO 2012/136474

(56) Entgegenhaltungen:
- EP-A2- 0 745 581
- WO-A1-94/29421

## Beschreibung

Die Erfindung betrifft ein Verfahren zur Herstellung von wässrigen Lösungen von Methylglycin-N,N-diessigsäure-trialkalimetallsalzen.

Die häufig als Komplexbildner in Reinigungsmitteln verwendeten Stoffe wie Aminopolyphosphonate, Polycarboxylate oder Aminopolycarboxylate, beispielsweise Ethylendiamintetraessigsäure (EDTA), sind entweder nur schwer biologisch abbaubar oder, wie Nitrilotriessigsäure (NTA), toxisch. Für NTA besteht Verdacht auf Nierenkanzerogenität.

Eine preiswerte Alternative hierzu stellt die α-Alanin-N,N-diessigsäure (Methylglycin-N,N-diessigsäure, im Folgenden abgekürzt als MGDA bezeichnet) dar, die ungiftig und biologisch leicht abbaubar ist.

Die Verwendung von MGDA und ihrer Salze als Komplexbildner sowie ihre Synthesen sind in verschiedenen Patentanmeldungen der BASF SE, beispielsweise in DE-A 43 19 935 oder in EP-A 0 745 582, beschrieben.

Eine wirtschaftliche und gleichzeitig umweltschonende Syntheseroute zur Herstellung von MGDA ist die Streckersynthese. Die Streckersynthese von MGDA ist beispielsweise in WO-A 1994/29421 beschrieben.

In der DE-A 20 27 972 wird die "saure" Variante der Strecker-Reaktion von Glycin, der einfachsten unsubstituierten α-Aminocarbonsäure, mit Formaldehyd und Blausäure beschrieben. Hierbei bildet sich aus Glycin das N,N-Bis(cyanmethyl)glycin, welches in hoher Reinheit isoliert werden kann. Nachteilig bei dem beschriebenen Verfahren ist der notwendige Einsatz von zusätzlicher Säure zur Erniedrigung des pH-Wertes sowie die Verwendung von relativ teurem reinem Glycin. Das sich dabei bildende Glycin-N,N-diacetonitril wird zum Einsatz als Vernetzer beschrieben. Die mögliche Verseifung zur Nitrilotriessigsäure ist nicht Gegenstand der DE-A 20 27 972.

Die Umsetzung von Alanin mittels Strecker-Reaktion zu MGDA wird erstmals in WO-A 1994/29421 beschrieben, MGDA wird hier nach Verseifung in hohen Ausbeuten mit hoher Reinheit gewonnen.

Die "alkalische" Variante der Strecker-Reaktion wird beispielsweise in der US-A 3 733 355 in allgemeiner Form dargestellt. Die dort aufgeführten Beispiele zeigen jedoch, dass immer ein hoher Anteil an Nebenprodukten, vor allem an unerwünschter Nitrilotriessigsäure (NTA), auftritt; dies lässt sich aus den Umsätzen von nur maximal ca. 89 % schießen.

Die EP-A 0 745 582 beschreibt eine einfache und wirtschaftliche Syntheseroute für Glycin-N,N-diessigsäuren wie MGDA ausgehend von preiswerten Ausgangsmaterialien möglichst ohne zwischengeschaltete Reinigungsschritte, wobei eine möglichst hohe Gesamtausbeute bei gleichzeitig hohen Produktreinheiten, mit niedrigen NTA-Gehalten, möglichst unter 2 Gew.-% angestrebt wird, durch Streckersynthese in wässrigem Medium bei einem pH-Wert von 0 bis 11 und anschließender Verseifung, wobei man als Ausgangsmaterial aus der technischen Synthese von Glycin-Derivaten oder deren Vorstufen oder von Iminodiacetonitril oder Iminodiessigsäure stammendes nicht gereinigtes, d.h. in der Regel nicht als Feststoff isoliertes oder beispielsweise durch Kristallisation von Nebenbestandteilen befreites Rohmaterial oder bei solchen Synthesen anfallende Mutterlaugen einsetzt.

Es war demgegenüber Aufgabe der vorliegenden Erfindung, ein weiter verbessertes Verfahren zur Herstellung von MGDA-Trialkalimetallsalzen durch Streckersynthese zur Verfügung zu stellen, das sich insbesondere durch eine weiter erhöhte Raum-Zeit-Ausbeute sowie weiter erniedrigte Gehalte an toxischen Nebenkomponenten, insbesondere an NTA, unter 0,1 Gew.-% NTA, bezogen auf eine 40 Gew.-%ige wässrige MGDA-trialkalimetallsalz-Lösung, kennzeichnet.

Die Aufgabe wird gelöst durch ein Verfahren zur Herstellung von wässrigen Lösungen von Methylglycin-N,N-diessigsäure-trialkalimetallsalzen durch Strecker-Synthese, ausgehend von einer wässrigen Lösung von α-Alanin, durch Umsetzung mit Formaldehyd und Blausäure zu α-Alanin-N,N-diacetonitril in einer Reaktionseinheit und Verseifen desselben mit einer Base zur wässrigen Lösung des entsprechenden Methylglycin-N,N-diessigsäure-trialkalimetallsalzes, das dadurch gekennzeichnet ist, dass man
- das α-Alanin teilneutralisiert und
- die Zugabe des Formaldehyds und der Blausäure für die Umsetzung zum α-Alanin-N,N-diacetonitril so steuert, dass die Konzentration an freier Blausäure in der wässrigen Reaktionsmischung zu jedem Zeitpunkt so begrenzt wird, dass Nebenreaktionen, insbesondere zu Formaldehydcyanhydrin, einschließlich Folgereaktionen von Formaldehydcyanhydrin sowie die Polymerisation von Blausäure nur insoweit stattfinden, dass die wässrige Lösung des Methylglycin-N,N-diessigsäure-trialkalimetallsalzes bei einer Konzentration von 40 Gew.-% des Methylglycin-N,N-diessigsäure-trialkalimetallsalzes aufweist, bezogen auf das Gesamtgewicht der wässrigen Methylglycin-N,N-diessigsäure-trialkalimetall-Lösung einen Nitrilotriessigsäure-trialkalimetalsalz-Gehalt von kleiner als 0,1 Gew.-%,
   dergestalt, dass die Konzentration an freier Blausäure in der flüssigen Reaktionsmischung zu keinem Zeitpunkt 10 Mol-% überschreitet, bezogen auf die Menge der zudosierten Blausäure, oder dergestalt,
   dass die maximale Konzentration an freier Blausäure in der flüssigen Reaktionsmischung 20 Mol-% freie Blausäure, bezogen auf die Menge des zudosierten teilneutralisierten α-Alanins, nicht überschreitet..

Die Erfinder haben erkannt, dass es möglich ist, durch Teilneutralisation der Aminosäure α-Alanin von deutlich erhöhten Konzentrationen dieses Einsatzstoffes in der wässrigen Lösung, die der Streckersynthese unterworfen wird, auszugehen, und somit die Raum-Zeit-Ausbeute deutlich zu erhöhen. Gegenüber der Löslichkeitsgrenze der freien Aminosäure α-Alanin bei Raumtemperatur von ca. 18 Gew.-%, bezogen auf das Gesamtgewicht der Lösung, können durch Teilneutralisation Konzentrationen an teilneutralisiertem α-Alanin in Wasser von bis zu 40 Gew.-% α-Alanin, oder auch von bis zu 50 Gew.-% α-Alanin, bezogen auf das Gesamtgewicht der wässrigen Lösung, erreicht werden, mit entsprechender Steigerung der Raum-Zeit-Ausbeute.

Würde jedoch die Streckersynthese in ansonsten bekannter Weise, jedoch ausgehend von teilneutralisiertem α-Alanin durchgeführt, so würde der höhere pH-Wert Nebenreaktionen, insbesondere solche, die zur Bildung des toxischen NTA führen, sowie die unerwünschte Polymerisation der Blausäure, die zu einer unerwünscht dunklen Farbe des Produktes führt, beschleunigen.

Überraschend wurde jedoch gefunden, dass diese unerwünschten Nebenreaktionen begrenzt werden können, so dass die Spezifikationsanforderungen für MGDA-Trialkalimetallsalz, insbesondere bezüglich NTA-Gehalt und Farbe, eingehalten werden, indem die Zugabe von Formaldehyd und Blausäure für die Umsetzung zum α-Alanin-N,N-diacetonitril so gesteuert wird, dass die Konzentration an freier Blausäure in der flüssigen Reaktionsmischung in geeigneter Weise begrenzt wird.

Die Spezifikationsanforderungen für MGDA-Trialkalimetallsalz bezüglich NTA-Gehalt und Farbe sind allgemein bekannt:
So soll die Menge an dem potentiell kanzerogenen NTA (Verdacht auf krebserzeugende Wirkung) in einer ca. 40 Gew.-%igen wässrigen MGDA-Na₃-Salz-Lösung unter 0,1 Gew.-% liegen (vergleiche z.B. "Technical Bulletin - Trilon^{®}M Liquid Chelating Agent", BASF 2009).

Die Produktspezifikation für Trilon®M flüssig ist bezüglich der Farbe ebenfalls allgemein bekannt und sieht eine Hazen-Farbzahl nach DIN EN1557 von maximal 350 vor (vergleiche "Technische Information Trilon^{®}M Marken", BASF Ausgabe April 2011, Seite 4).

Bevorzugt wird die Blausäure dergestalt in die flüssige Reaktionsmischung zudosiert, dass die Konzentration an freier Blausäure in der flüssigen Reaktionsmischung zu keinem Zeitpunkt 10 Mol-%, bezogen auf die Menge der zudosierten Blausäure, bevorzugt 5 Mol-%, bezogen auf die Menge der zudosierten Blausäure, beträgt.

Die maximal zulässige Konzentration an freier Blausäure in der flüssigen Reaktionsmischung kann auch auf das zudosierte teilneutralisierte α-Alanin bezogen werden und soll dann 20 Mol-%, bevorzugt 10 Mol-% freie Blausäure, bezogen auf die Menge des zudosierten teilneutralisierten α-Alanins nicht überschreiten.

Die Umsetzung von teilneutralisiertem α-Alanin mit Blausäure und Formaldehyd durch Streckersynthese erfordert ein stöchiometrisches Molverhältnis von 1 Mol α-Alanin zu jeweils 2 Mol Blausäure und 2 Mol Formaldehyd. In der Praxis wird in der Regel bei einem Molverhältnis von α-Alanin zu Blausäure als auch von α-Alanin zu Formaldehyd von jeweils 1,95 bis 2,4, bevorzugt 2,0 bis 2,2, gearbeitet.

In einer bevorzugten Ausführungsform des Verfahrens wird die Zugabe des Formaldehyds und der Blausäure für die Umsetzung zum α-Alanin-N,N-diacetonitril so gesteuert, dass die Konzentration an freier Blausäure in der wässrigen Reaktionsmischung zu jedem Zeitpunkt so begrenzt wird, dass Nebenreaktionen, insbesondere zu Formaldehydcyanhydrin, einschließlich Folgereaktionen von Formaldehydcyanhydrin sowie die Polymerisation von Blausäure nur insoweit stattfinden, dass die wässrige Lösung des Methylglycin-N,N-diessigsäure-trialkalimetallsalzes eine Hazen-Farbzahl von kleiner als 800, bevorzugt von kleiner als 600, besonders bevorzugt von kleiner als 500, aufweist.

In einer weiteren bevorzugten Ausführungsform wird die wässrige Lösung des Methylglycin-N,N-diessigsäure-trialkalimetallsalzes in einem weiteren Verfahrensschritt einer Nachbleiche unterzogen, unter Erhalt einer wässrigen Lösung des Methylglycin-N,N-diessigsäure-trialkalimetallsalzes mit einer Hazen-Farbzahl von kleiner als 350, bevorzugt von kleiner als 300.

Die Nachbleiche (=Finish) kann durch eine chemische und/oder eine physikalische Operation erfolgen. Die chemische Operation kann oxidativ sein, beispielsweise mit Wasserstoffperoxid oder Luft, oder reduktiv, beispielsweise mit Natriumdithionit oder Natriumhydrid. Physikalisch kann die Nachbleiche durch Absorption von Farbkomponenten, zum Beispiel an Aktivkohle, durchgeführt werden.

Besonders bevorzugt wird die Nachbleiche unter Verwendung von wässrigen Wasserstoffperoxid-Lösungen durchgeführt.

Die Nachbleiche der wässrigen MGDA-Trialkalimetallsalz-Lösungen mit Wasserstoffperoxid kann abschnittsweise oder kontinuierlich durchgeführt werden.

So kann zum Beispiel die Wasserstoffperoxid-Lösung unter effizientem Rühren zu einer vorgelegten MGDA-Trialkalimetallsalz-Lösung in einen Rührkessel oder in einen umgepumten Produktkreislauf zu dosiert werden. Mengen, Temperaturen und Verweilzeiten sind bevorzugt wie folgt:
Es genügen in der Regel sehr geringe Wasserstoffperoxidmengen, insbesondere 1 bis 5 kg, bevorzugt 1 bis 3 kg Wasserstoffperoxid (gerechnet als 100% H₂O₂) pro 1000 Liter 40 %-ige MGDA-Trialkalimetallsalz-Lösung (entsprechend ca. 1300 kg)

Bevorzugt wird 10 bis 50 %-ige wässrige Wasserstoffperoxid-Lösung verwendet, besonders bevorzugt 30%-ige wässrige Wasserstoffperoxid-Lösung, sogenanntes Perhydrol.

Bevorzugt sind Temperaturen zwischen 20 und 80°C, weiter bevorzugt zwischen 30 und 70°C, besonders bevorzugt zwischen 40 und 65°C. Bevorzugte Verweilzeiten sind zwischen 10 und 180 Minuten, besonders bevorzugt zwischen 15 und 120 Minuten.

In einer weiteren Ausführungsvariante wird von kristallinem α-Alanin als Einsatzstoff ausgegangen, das in Wasser aufgelöst oder suspendiert wird und anschließend in der oben beschriebenen Weise teilneutralisiert und zum MGDA-Trialkalimetallsalz umgesetzt wird.

Die Teilneutralisation des α-Alanins kann insbesondere mit Natriumhydroxid oder Kaliumhydroxid oder einer Mischung aus Natriumhydroxid und Kaliumhydroxid durchgeführt werden. Insbesondere kann die Neutralisation des α-Alanins auf einen Neutralisationsgrad von 40 bis 90 %, bevorzugt von 50 bis 85 %, besonders bevorzugt von 60 bis 80 % durchgeführt werden. Hierbei werden konzentrierte wässrige Lösungen erhalten, die bevorzugt 20 bis 50 Gew.-% α-Alanin, weiter bevorzugt 25 bis 40 Gew.-% α-Alanin, bezogen auf das Gesamtgewicht der wässrigen Lösung, enthalten.

In einer ersten Verfahrensvariante wird teilneutralisiertes α-Alanin in wässriger Lösung mit Formaldehyd und Blausäure derart zu einer wässrigen Lösung von α-Alanin-N,N-diacetonitril umgesetzt, dass Blausäure in zeitlichem Nachgang gegenüber den beiden anderen Reaktanden, teilneutralisiertes α-Alanin und Formaldehyd, in die flüssige Reaktionsmischung eingebracht wird.

Hierbei kann insbesondere sowohl abschnittsweise (Semi-Batch) als auch kontinuierlich gefahren werden.

Nach einer ersten bevorzugten Verfahrensvariante, in Semi-Batch-Fahrweise, wird teilneutralisiertes wässriges α-Alanin in einem Reaktor vorgelegt und Formaldehyd sowie gegenüber Formaldehyd zeitlich langsamer Blausäure parallel zudosiert.

In einer zweiten Verfahrensvariante wird teilneutralisiertes α-Alanin zunächst mit der vollständigen für die Umsetzung erforderlichen Menge Formaldehyd oder einer Teilmenge desselben umgesetzt, und das resultierende Reaktionsgemisch anschließend mit der für die Umsetzung erforderlichen Blausäure allein oder mit Blausäure und der restlichen Formaldehydmenge parallel zudosiert.

In einer weiteren bevorzugten Verfahrensvariante wird die Streckersynthese kontinuierlich durchgeführt, bevorzugt in einer Kaskade von zwei oder mehreren hintereinander geschalteten Reaktionszonen.

Die zwei oder mehreren hintereinander geschalteten Reaktionszonen können jeweils unterschiedliche Reaktionszonen in einem einzigen Reaktor oder einzelne Reaktoren sein.

Bevorzugt wird in einer ersten Reaktionszone teilneutralisiertes α-Alanin getrennt oder vorvermischt mit der gesamten für die Umsetzung erforderlichen Formaldehydmenge allein oder zusätzlich mit einer Teilmenge der für die Umsetzung erforderlichen Blausäure parallel zudosiert. In einer nachfolgenden Reaktionszone wird das resultierende Reaktionsgemisch aus der ersten Reaktionszone mit der gesamten für die Umsetzung erforderlichen Blausäuremenge oder mit der restlichen Blausäuremenge parallel zudosiert.

Die Verseifung der durch Streckersynthese erhaltenen wässrigen Lösungen von α-Alanin-N,N-diacetonitril wird vorteilhaft in der Weise durchgeführt, dass zunächst mit Natron- oder Kalilauge oder einem Gemisch aus Natron- und Kalilauge bei einer Temperatur im Bereich von 20 bis 80 °C, bevorzugt bei einer Temperatur im Bereich von 30 bis 70 °C, und anschließend bei Temperaturen ≥ 90 °C zu wässrigen MGDA-Trialkalimetallsalz-Lösungen verseift wird. Hierbei wird während der Verseifung bei Temperaturen ≥ 90 °C gleichzeitig die Reaktionslösung von Ammoniak befreit.

Die Verseifung kann ebenfalls sowohl abschnittsweise (Semibatch) als auch kontinuierlich durchgeführt werden.

In einer bevorzugten Verfahrensvariante wird vom L-Enantiomeren des α-Alanins ausgegangen.

Das L-Enantiomere des α-Alanin ist preiswert verfügbar und führt durch Streckersynthese, durch Umsetzung mit Blausäure und Formaldehyd in wässriger Lösung zum L-α-Alanin-N,N-diacetonitril und Verseifen desselben mit einer Base zu wässrigen Lösungen, die nicht nur eine höhere Sättigungskonzentration des L-Enantiomeren im Vergleich zum D,L-Racemat der MGDA-Trialkalimetallsalze aufweisen, sondern die auch durch Kristallisation einen gut kristallisierenden Feststoff ergeben.

Die Vorteile der höheren Löslichkeit von L-MGDA-Trialkalimetallsalz im Vergleich zum Racemat liegen darin, dass die Synthese, die Lagerung, der Verkauf und der Transport von höher konzentrierten Lösungen möglich ist, und dadurch Kosteneinsparungen durch beispielsweise verbesserte Raumausbeuten, kleinvolumigere Synthese- und Lagerbehälter sowie reduzierte Transport- oder Energiekosten, z.B. bei Sprühtrocknungsverfahren, erreicht werden.

Die Erfindung wird im Folgenden anhand von Ausführungsbeispielen näher erläutert.

### Vergleichsbeispiel 1

### (Semibatch; Neutralisationsgrad 0, Alanin-Konzentration ca. 18%; Überschüsse an HCHO und HCN jeweils 0,03 Äquivalente)

Zu einer Lösung von 89 g (1,0 mol) α-Alanin in 405 g Wasser (ca. 18%ig) wurden bei 30°C unter Kühlen parallel innerhalb von 1 Stunde 203 g 30%iger Formaldehyd (2,03 mol) und 54,8 g (2,03 mol) Blausäure dosiert. Man ließ anschließend 1 Stunde bei 30°C nachrühren. Gesamt-Cyanid, d.h. Summe aus freier, nicht umgesetzter HCN und an im Formaldehydcyanhydrin gebundenen Cyanid 0,32% (Analytik = potentiometrische Titration); HCN-Umsatz aus potentiometrischer Titration der freien HCN 96%, pH 1,7.

Man legte 245 g 50%ige Natronlauge (3.06 mol) im Rührkolben vor und dosierte die obige α-Alanin-N,N-diacetonitril-(ADAN-)Lösung innerhalb von 1 Stunde bei 27 bis 36°C unter Kühlen zu. Anschließend rührte man 60 Minuten bei ca. 30°C weiter. Dann erhitzte man auf 95 bis 102°C und vervollständigte die Verseifung innerhalb von ca. 3 Stunden.

Es resultierten 635 g einer gemäß HPLC-Auswertung 39,8%igen Lösung MGDA-tri-Natriumsalz, mit einem NTA-Na3-Gehalt von 0,20%, sowie einer Hazen-Farbzahl von 850, was den Spezifikationsanforderungen nicht entspricht, und einer Ausbeute von 93,2%.

### Vergleichsbeispiel 2

### (Semibatch; wie Vergleichsbeispiel 1, aber mit 100%igem Alanin-Neutralisationsgrad, Alanin-Konzentration ca. 40%)

89 g (1,0 mol) α-Alanin wurden in 55 g Wasser vorgelegt. Unter Kühlen wurde mit 80 g 50%iger Natronlauge (1,0 mol) vollständig neutralisiert (ca. 40%iges Alanin); Start-pH-Wert 13,7.

Bei ca. 30°C wurden unter Kühlen innerhalb von 1 Stunde 203 g 30%iger Formaldehyd (2,03 mol) und 54,8 g (2,03 mol) Blausäure dosiert. Man ließ anschließend 1 Stunde bei 30 °C nachrühren.

Analytik der resultierenden ADAN-Lösung: Gesamt-Cyanid 2,86%, HCN-Umsatz 60,5%, End-pH 8,0.

165 g 50%ige Natronlauge (2,06 mol) wurden vorgelegt. Hierzu wurde bei 30-35°C obige ADAN-Lösung innerhalb von 1 Stunde unter Kühlen dosiert. Anschließend ließ man 60 Minuten bei ca. 30°C weiter rühren. Dann erhitzte man auf 95-102 °C und vervollständigte die Verseifung innerhalb von ca. 4 Stunden. Es resultierten 482 g einer gemäß HPLC-Auswertung 38,6%igen Lösung von MGDA-tri-Natriumsalz, mit einem NTA-Na3-Gehalt von 5,1% (nicht spezifikationsgerecht). Ausbeute an MGDA-Na3: 68,6%, Hazen-Farbzahl deutlich größer als 1000.

### Vergleichsbeispiel 3

### (Semibatch; wie Vergleichsbeispiel 2, aber mit 85%igem Alanin-Neutralisationsgrad, Alanin-Konzentration ca. 42%)

89 g (1,0 Mol) α-Alanin wurden in 55 g Wasser vorgelegt. Unter Kühlen wurde mit 68 g 50%iger Natronlauge (0,85 Mol) teilneutralisiert. Start-pH: 11,5. Bei ca. 30°C wurden unter Kühlen innerhalb von 1 Stunde 203 g 30%iger Formaldehyd (2,03 Mol) und 54,8 g (2,03 Mol) Blausäure dosiert. Man ließ anschließend 1 Stunde bei 30°C nachrühren.

Analytik der resultierenden ADAN-Lösung: Gesamt-Cyanid 0,24%, HCN-Umsatz 98%. End-pH 4,5.

Die Verseifung wurde wie in Vergleichsbeispiel 2 mit 176,8 g 50%iger Natronlauge (2,21 Mol) durchgeführt.

Es resultierten 657 g einer 40,3%igen MGDA-Na3-Lösung mit einem NTA-Na3-Gehalt von 0,12%.

Ausbeute an MGDA-Na3: 97,7% bezüglich Alanin, Hazen-Farbzahl > 1000.

### Vergleichsbeispiel 4

### (Semibatch, wie Beispiel 1, aber mit Alanin-Neutralisationsgrad 65%, Alanin-Konzentration ca. 30%)

133,5 g (1,5 Mol) α-Alanin wurden in 230 g Wasser vorgelegt. Unter Kühlen wurde mit 78 g 50%iger Natronlauge (0,975 Mol) teilneutralisiert. Bei 40°C wurden innerhalb von 1 h parallel 82,3 g (3,05 Mol) HCN und 305 g 30%iger Formaldehyd (3,05 Mol) dosiert, 1 h bei 40°C nachgerührt.

Analytik: Gesamt-Cyanid 0,23 %. HCN-Umsatz 98%. End-pH 3,9.

Die Verseifung wurde analog Vergleichsbeispiel 2 mit 288,8 g 50%iger Natronlauge (3,61 mol) durchgeführt.

Man erhielt 903 g einer 43,2%igen MGDA-Na3-Lösung mit einem NTA-Na3-Gehalt von 0,15%.

Ausbeute an MGDA-Na3: 96%, Hazen-Farbzahl 900.

### Vergleichsbeispiel 5

### (Semikontinuierliche Verfahrensführung: Parallele Dosierung aller 3 Komponenten mit 18%igem Alanin, Neutralisationsgrad 0)

Allgemeine Durchführung: Eine geringe Wassermenge wurde im Rührkessel vorgelegt. Anschließend wurden parallel die 3 Reaktanden innerhalb von 60 Minuten bei 40°C dosiert. 1 Stunde Nachreaktion.

Analog wurde zur Verseifung eine kleine NaOH-Menge (ca. 10%) vorgelegt und der große NaOH-Rest sowie die ADAN-Lösung parallel bei ca. 30-35°C innerhalb von 1 Stunde dosiert. Nachreaktion und Endverseifung erfolgten wie unter Semibatch-Bedingungen. MGDA-Na3-Ausbeute: 92,1% bezüglich Alanin; NTA-Na3-Gehalt einer 40,1%igen MGDA-Na3-Lösung: 0,24%, Hazen-Farbzahl 950.

### Vergleichsbeispiel 6

### (Semikontinuierliche Verfahrensführung, wie Vergleichsbeispiel 5, aber mit 30%iger Alanin- Konzentration, Neutralisationsgrad ca. 70%)

MGDA-Na3-Ausbeute: 92,7% bezüglich Alanin; NTA-Na3-Gehalt einer 39,9%igen MGDA-Na3-Lösung: 0,17%, Hazen-Farbzahl >1000.

### Vergleichsbeispiel 7

### (Kontinuierliche Verfahrensführung / Rührkesselkaskade - ohne Blausäure-Split)

Die kontinuierliche Herstellung einer ca. 40%igen MGDA-Na3-Lösung erfolgte in einer Anlage bestehend aus jeweils 3 Rührkesseln bei 40°C in der Nitril- (R1, R2 und R3) und 40°C in der Verseifungsstufe (R4, R5, R6). Anschließend erfolgte eine Vervollständigung der Verseifung in Rührkessel R7 bei 105-110°C sowie eine abschließende Ammoniakstrippung. HCN, Formaldehyd und eine Lösung aus Alanin (65% teilneutralisiert mit Natronlauge, 30% Gehalt bezogen auf Alanin) wurden in R1 dosiert, die Lauge in R4. Die Molverhältnisse der Einsatzstoffe wurden wie im Vergleichsbeispiel 4 gewählt. Die Dosierung erfolgte so, dass die Verweilzeiten in R1-R2-R3 bei 55-80 Minuten, in R3-R6 bei 200-280 Minuten sowie in R7 bei 150-200 Minuten liegen. Das Produkt hat typischerweise einen NTA-Na3-Gehalt von 0,25-0,40% und Hazen-Farbzahlen > 1000.

MGDA-Na3-Ausbeute: 92-93% bezüglich Alanin.

### Beispiel 1

### (Semibatch, wie Vergleichsbeispiel 4, aber mit schnellerer Formaldehyd-Dosierung versus HCN)

Im Unterschied zu Vergleichsbeispiel 4 wurde Formaldehyd in 30 Minuten, HCN in 60 Minuten zudosiert.

MGDA-Na3-Ausbeute: 98,1% bezüglich Alanin; NTA-Na3-Gehalt in der 40,4%igen MGDA-Na3-Lösung: 0,06%, Hazen-Farbzahl 320.

### Beispiel 2

### (mit Nachbleiche)

Zu der nach Beispiel 1 erhaltenen Lösung wurden unter kräftigem Rühren 10 g einer 30%igen Wasserstoffperoxid-Lösung innerhalb von 15 Minuten bei ca. 60°C dosiert. Anschließend rührte man ca. 30 Minuten bei 60°C weiter. Die resultierende Lösung wies eine Hazen-Farbzahl von 180 auf.

### Beispiel 3

### (Semibatch, Alanin-Konzentration 30%, Neutralisationsgrad ca. 70%)

89 g (1,0 mol) α-Alanin wurden in 150 g Wasser vorgelegt. Unter Kühlen wurde mit 56 g 50%iger Natronlauge (0,7 mol) teilneutralisiert.

Bei 40°C wurden parallel bei ca. 40°C 203 g 30%iger Formaldehyd (2,03 mol) innerhalb von 60 Minuten und 54,8 g (2,03 mol) Blausäure innerhalb von 90 Minuten dosiert. 30 Minuten wurde anschließend bei 40°C nachgerührt.

Analytik der resultierenden ADAN-Lösung: Gesamt-Cyanid 0,29%, HCN-Umsatz 99%, End-pH 4,1

189 g 50%ige Natronlauge (2,36 mol) wurden vorgelegt. Hierzu dosierte man bei 45-50°C obige ADAN-Lösung innerhalb von 1 Stunde zu. Anschließend ließ man 60 Minuten bei 50°C weiter rühren.

Dann vervollständigte man bei 95-102°C die Verseifung sowie die Ammoniak-Destillation innerhalb von ca. 3 Stunden.

Es resultierten 665 g einer ca. 39,5%igen MGDA-Na3-Lösung.

Ausbeute: 97,0% - NTA-Na3-Gehalt 0,08% - Hazen-Farbzahl 340.

### Beispiel 4

### (Semikontinuierliche Verfahrensführung, wie Vergleichsbeispiel 4, aber mit schnellerer Dosierung von Alanin und Formaldehyd in 30 Minuten, Blausäure-Zugabe in 60 Minuten)

Bestes Ergebnis: MGDA-Na3-Ausbeute: 97,6% - NTA-Na3-Gehalt einer 40,3%igen ADA- Na3-Lösung: 0,03%, Hazen-Farbzahl 330.

### Beispiel 5

Die nach Beispiel 4 erhaltene Lösung wurde unter den Bedingungen von Beispiel 2 gebleicht. Es resultierte eine Hazen-Farbzahl von 150.

### Beispiel 6

### (Semikontinuierliche Verfahrensführung, wie Vergleichsbeispiel 4, aber mit Blausäure-Split 30%)

Ca. 30%iges Alanin mit Neutralisationsgrad 70%, Formaldehyd und nur 70% der gesamten Blausäure-Menge wurden innerhalb von 60 Minuten parallel bei 40°C dosiert. Anschließend wurde das resultierende Reaktionsgemisch ohne Nachreaktion abgelassen und sofort parallel mit den restlichen 30% der Blausäure innerhalb von 60 Minuten bei 40°C dosiert. Man ließ 30 Minuten bei 40°C weiter rühren.

Die Verseifung erfolgte wie in Vergleichsbeispiel 5.

MGDA-Na3-Ausbeute: 96,6% - NTA-Na3-Gehalt: 0,07%, Hazen-Farbzahl 370.

### Beispiel 7

### (Semikontinuierliche Reaktionsführung, wie Beispiel 6, mit 30%iger Alanin-Konzentration, Neutralisationsgrad ca. 70%, aber mit Blausäure-Split 50%)

MGDA-Na3-Ausbeute: 97,3% - NTA-Na3-Gehalt einer 39,9%igen MGDA-Na3-Lösung: 0,05%, Hazen-Farbzahl 320.

### Beispiel 8

### (Kontinuierliche Verfahrensführung / Rührkesselkaskade - mit Blausäure-Split)

Die kontinuierliche Herstellung einer ca. 40%igen MGDA-Na3-Lösung erfolgte wie in Vergleichsbeispiel 7, wobei aber die Dosierung der HCN auch in den R2 erfolgte und die dosierte Menge HCN im Verhältnis 4:1 zwischen R1 und R2 aufgeteilt wurde. Das Produkt hat typischerweise einen NTA-Na3-Gehalt von <0,1% und Hazen-Farbzahlen von 450-650.

MGDA-Na3-Ausbeute: 97-98,5% bezüglich Alanin.

### Beispiel 9

### (mit Nachbleiche)

Die in Beispiel 8 erhaltene Lösung wurde anschließend in einer Verweilzeitstrecke durch Dosieren von 30%igem Wasserstoffperoxid (ca. 5 Liter pro 1 m³ Lösung) bei 40-50°C und Vervollständigung der Reaktion in einem Verweilzeitbehälter gebleicht.

Es resultierten typischerweise Hazen-Farbzahlen von <300.

### Beispiel 10:

### Herstellung einer ca. 50 gew.-%igen L-MGDA-Na3-Lösung

### (analog zu Beispiel 8, jedoch wurde als Alaninquelle L-α-Alanin eingesetzt)

In der letzten Stufe der Verseifung bei 95-102°C wurde der gebildete Ammoniak und soviel Wasser abdestilliert, dass abschließend eine ca. 50 gew.-%ige Lösung von L-MGDA-Na3-Salz resultierte.

Ausbeute an L-MGDA-Na3: 97,0% bezogen auf L-Alanin. NTA-Na3-Gehalt: 0,08%. Hazen-Farbzahl 270.

Es wird somit eine farbhelle, hochkonzentrierte (50 Gew.-%ige) Komplexbildnerlösung mit sehr niedrigem Restgehalt an NTA erhalten.

## Patentansprüche

1. Verfahren zur Herstellung von wässrigen Lösungen von Methylglycin-N,N-diessigsäure-trialkalimetallsalzen durch Strecker-Synthese, ausgehend von einer wässrigen Lösung von α-Alanin, durch Umsetzung mit Formaldehyd und Blausäure zu α-Alanin-N,N-diacetonitril in einer Reaktionseinheit und Verseifen desselben mit einer Base zur wässrigen Lösung des entsprechenden Methylglycin-N,N-diessigsäure-trialkalimetallsalzes, **dadurch gekennzeichnet, dass** man
- das α-Alanin teilneutralisiert und
- die Zugabe des Formaldehyds und der Blausäure für die Umsetzung zum α-Alanin-N,N-diacetonitril so steuert, dass die Konzentration an freier Blausäure in der wässrigen Reaktionsmischung zu jedem Zeitpunkt so begrenzt wird, dass Nebenreaktionen, insbesondere zu Formaldehydcyanhydrin, einschließlich Folgereaktionen von Formaldehydcyanhydrin sowie die Polymerisation von Blausäure nur insoweit stattfinden, dass die wässrige Lösung des Methylglycin-N,N-diessigsäure-trialkalimetallsalzes einen Nitrilotriessigsäuretrialkalimetallsalz-Gehalt von kleiner als 0,1 Gew.-% bei einer Konzentration von 40 Gew.-% des Methylglycin-N,N-diessigsäure-trialkalimetallsalzes aufweist, bezogen auf das Gesamtgewicht der wässrigen Methylglycin-N,N-diessigsäure-trialkalimetallsalz-Lösung,
dergestalt, dass die Konzentration an freier Blausäure in der flüssigen Reaktionsmischung zu keinem Zeitpunkt 10 Mol-% beträgt, bezogen auf die Menge der zudosierten Blausäure, oder dergestalt,
dass die maximale Konzentration an freier Blausäure in der flüssigen Reaktionsmischung 20 Mol-% freie Blausäure, bezogen auf die Menge des zudosierten teilneutralisierten α-Alanins, nicht überschreitet.

2. Verfahren nach Anspruch 1, **dadurch gekennzeichnet, dass** man die Blausäure in zeitlichem Nachgang gegenüber dem teilneutralisierten α-Alanin und dem Formaldehyd in die Reaktionseinheit einbringt.

3. Verfahren nach Anspruch 1 oder 2, **dadurch gekennzeichnet, dass** man
nach einem Semibatch-Verfahren vorgeht und teilneutralisiertes wässriges α-Alanin in der Reaktionseinheit vorlegt und Formaldehyd und Blausäure parallel zudosiert, wobei die Zudosierung der Blausäure gegenüber dem Formaldehyd zeitlich langsamer erfolgt, oder
das teilneutralisierte α-Alanin mit Formaldehyd und Blausäure kontinuierlich umsetzt, wobei die Umsetzung in einer Kaskade von zwei oder mehreren hintereinander geschalteten Reaktionszonen durchgeführt wird.

4. Verfahren nach einem der Ansprüche 1 bis 3, **dadurch gekennzeichnet, dass** man die wässrige Lösung von α-Alanin erhält, indem man von kristallinem α-Alanin als Einsatzstoff ausgeht und dieses in Wasser auflöst oder suspendiert und mit einer Base teilneutralisiert.

5. Verfahren nach einem der Ansprüche 1 bis 4, **dadurch gekennzeichnet, dass** man die Teilneutralisation des α-Alanins mit Natriumhydroxid oder Kaliumhydroxid oder einem Gemisch aus Natron- und Kalilauge auf einen Neutralisationsgrad von 40 bis 90% unter Erhalt einer konzentrierten wässrigen Lösung durchführt, enthaltend 20 bis 50 Gew.-% Alanin, bezogen auf das Gesamtgewicht der wässrigen Lösung.

6. Verfahren nach einem der Ansprüche 1 bis 5, **dadurch gekennzeichnet, dass** man das Verfahren als Semi-Batch-Verfahren durchführt, indem teilneutralisiertes wässriges α-Alanin in der Reaktionseinheit vorgelegt wird und Formaldehyd und Blausäure parallel zudosiert werden, wobei die Zudosierung der Blausäure gegenüber dem Formaldehyd zeitlich langsamer erfolgt.

7. Verfahren nach einem der Ansprüche 1 bis 6, **dadurch gekennzeichnet, dass** die Umsetzung des teilneutralisierten α-Alanins mit Formaldehyd und Blausäure kontinuierlich in einer Kaskade von zwei oder mehreren hintereinander geschalteten Reaktionszonen durchgeführt wird.

8. Verfahren nach Anspruch 7, **dadurch gekennzeichnet, dass** die zwei oder mehreren hintereinander geschalteten Reaktionszonen jeweils einzelne Reaktoren sind.

9. Verfahren nach Anspruch 8, **dadurch gekennzeichnet, dass** die zwei oder mehreren hintereinander geschalteten Reaktionszonen jeweils unterschiedliche Zonen innerhalb eines einzigen Reaktors sind.

10. Verfahren nach Anspruch 9, **dadurch gekennzeichnet, dass** in die erste Reaktionszone teilneutralisiertes α-Alanin getrennt oder vorvermischt mit der gesamten für die Umsetzung erforderlichen Formaldehydmenge allein oder zusätzlich mit einer Teilmenge der für die Umsetzung erforderlichen Blausäure parallel zudosiert und in einer nachfolgenden Reaktionszone zu dem aus der ersten Reaktionszone resultierenden Reaktionsgemisch die Gesamtmenge der für die Umsetzung erforderlichen Blausäure oder die restliche Blausäuremenge parallel oder vorvermischt zudosiert wird.

11. Verfahren nach einem der Ansprüche 1 bis 10, **dadurch gekennzeichnet, dass** man die Verseifung der durch Streckersynthese erhaltenen wässrigen Lösung von α-Alanin-N,N-Diacetonitril in der Weise durchführt, dass man zunächst mit Natron- oder Kalilauge oder einem Gemisch aus Natron- und Kalilauge bei 20 bis 80°C, und anschließend bei Temperaturen von ≥ 90°C unter Freisetzung von Ammoniak zu wässrigen Methylglycin-N,N-diessigsäure-trialkalimetallsalzen umsetzt.

12. Verfahren nach einem der Ansprüche 1 bis 11, **dadurch gekennzeichnet, dass** die wässrige Lösung von α-Alanin eine wässrige Lösung von L-α-Alanin ist.

## Claims

1. A process for the preparation of aqueous solutions of methylglycine-N,N-diacetic acid trialkali metal salts by Strecker synthesis, starting from an aqueous solution of α-alanine, by reaction with formaldehyde and hydrocyanic acid to give α-alanine-N,N-diacetonitrile in one reaction unit and saponification thereof with a base to give the aqueous solution of the corresponding methylglycine-N,N-diacetic acid trialkali metal salt, wherein
- the α-alanine is partially neutralized and
- the addition of the formaldehyde and the hydrocyanic acid for the conversion to the α-alanine-N,N-diacetonitrile is controlled such that the concentration of free hydrocyanic acid in the aqueous reaction mixture at any time is limited such that secondary reactions, in particular to give formaldehyde cyanohydrin, including consecutive reactions of formaldehyde cyanohydrin, and also the polymerization of hydrocyanic acid take place only insofar as the aqueous solution of the methylglycine-N,N-diacetic acid trialkali metal salt has a nitrilotriacetic acid trialkali metal salt content of less than 0.1% by weight at a concentration of 40% by weight of the methylglycine-N,N-diacetic acid trialkali metal salt, based on the total weight of the aqueous methylglycine-N,N-diacetic acid trialkali metal salt solution,
such that the concentration of free hydrocyanic acid in the liquid reaction mixture at no time is 10 mol%, based on the amount of hydrocyanic acid metered in, or such that
the maximum concentration of free hydrocyanic acid in the liquid reaction mixture does not exceed 20 mol% of free hydrocyanic acid, based on the amount of partially neutralized α-alanine metered in.

2. The process according to claim 1, wherein the hydrocyanic acid is introduced into the reaction unit in a later operation compared with the partially neutralized α-alanine and the formaldehyde.

3. The process according to claim 1 or 2, wherein the process is carried out as a semi-batch process by introducing partially neutralized aqueous α-alanine as initial charge in the reaction unit, and metering in formaldehyde and hydrocyanic acid in parallel, where the metered addition of the hydrocyanic acid takes place more slowly in terms of time compared with the formaldehyde, or the partially neutralized α-alanine is reacted continuously with formaldehyde and hydrocyanic acid, where the reaction is carried out in a cascade of two or more reaction zones connected in series.

4. The process according to any one of claims 1 to 3, wherein the aqueous solution of α-alanine is obtained by using crystalline α-alanine as feed material and dissolving or suspending it in water and partially neutralizing it with a base.

5. The process according to any one of claims 1 to 4, wherein the partial neutralization of the α-alanine is carried out with sodium hydroxide or potassium hydroxide or a mixture of sodium hydroxide solution and potassium hydroxide solution to a degree of neutralization of from 40 to 90% to give a concentrated aqueous solution comprising 20 to 50% by weight of alanine, based on the total weight of the aqueous solution.

6. The process according to any one of claims 1 to 5, wherein the process is carried out as a semi-batch process by introducing partially neutralized aqueous α-alanine as initial charge in the reaction unit, and metering in formaldehyde and hydrocyanic acid in parallel, where the metered addition of the hydrocyanic acid takes place more slowly in terms of time compared with the formaldehyde.

7. The process according to any one of claims 1 to 6, wherein the reaction of the partially neutralized α-alanine with formaldehyde and hydrocyanic acid is carried out continuously in a cascade of two or more reaction zones connected in series.

8. The process according to claim 7, wherein the two or more reaction zones connected in series are in each case individual reactors.

9. The process according to claim 8, wherein the two or more reaction zones connected in series are in each case different zones within a single reactor.

10. The process according to claim 9, wherein partially neutralized α-alanine, separately or premixed with the entire amount of formaldehyde required for the reaction, on its own or additionally with a part amount of the hydrocyanic acid required for the reaction, is metered into the first reaction zone in parallel and, in a subsequent reaction zone, the entire amount of the hydrocyanic acid required for the reaction or the remaining amount of hydrocyanic acid, in parallel or premixed, is metered into the reaction mixture resulting from the first reaction zone.

11. The process according to any one of claims 1 to 10, wherein the saponification of the aqueous solution of α-alanine-N,N-diacetonitrile obtained by Strecker synthesis is carried out such that firstly reaction is carried out with sodium hydroxide solution or potassium hydroxide solution or a mixture of sodium hydroxide solution and potassium hydroxide solution at 20 to 80°C, and then at temperatures of ≥ 90°C, with release of ammonia to give aqueous methylglycine-N,N-diacetic acid trialkali metal salts.

12. The process according to any one of claims 1 to 11, wherein the aqueous solution of α-alanine is an aqueous solution of L-α-alanine.

## Revendications

1. Procédé pour la préparation de solutions aqueuses de sels tri-métalliques alcalins de l'acide méthylglycine-N,N-diacétique par synthèse de Strecker, partant d'une solution d'α-alanine, par transformation avec du formaldéhyde et de l'acide cyanhydrique en α-alanine-N,N-diacétonitrile dans une unité de réaction et saponification de celui-ci avec une base en solution aqueuse du sel tri-métallique alcalin de l'acide méthylglycine-N,N-diacétique correspondant, **caractérisé en ce que**
- on neutralise partiellement l'α-alanine et
- on régule l'addition du formaldéhyde et de l'acide cyanhydrique pour la transformation en α-alanine-N,N-diacétonitrile de manière telle que la concentration en acide cyanhydrique libre dans le mélange réactionnel aqueux est limitée à tout moment de manière telle que des réactions secondaires, en particulier en formaldéhydecyanhydrine, y compris les réactions consécutives de la formaldéhydecyanhydrine ainsi que la polymérisation d'acide cyanhydrique n'ont lieu que dans une mesure telle que la solution aqueuse du sel tri-métallique alcalin de l'acide méthylglycine-N,N-diacétique présente une teneur en sel tri-métallique alcalin de l'acide nitrilotriacétique inférieure à 0,1% en poids à une concentration de 40% en poids en sel tri-métallique alcalin de l'acide méthylglycine-N,N-diacétique par rapport au poids total de la solution aqueuse de sel tri-métallique alcalin de l'acide méthylglycine-N,N-diacétique,
de manière telle que la concentration en acide cyanhydrique libre dans le mélange réactionnel liquide n'est à aucun moment de 10% en mole, par rapport à la quantité d'acide cyanhydrique dosé ou de manière telle
que la concentration maximale en acide cyanhydrique libre dans le mélange réactionnel liquide ne dépasse par 20% en mole d'acide cyanhydrique libre, par rapport à la quantité d'α-alanine partiellement neutralisée dosée.

2. Procédé selon la revendication 1, **caractérisé en ce qu'**on introduit l'acide cyanhydrique postérieurement dans le temps par rapport à l'α-alanine partiellement neutralisée et au formaldéhyde dans l'unité de réaction.

3. Procédé selon la revendication 1 ou 2, **caractérisé en ce qu'**on procède selon un procédé semi-continu et on dispose l'α-alanine aqueuse partiellement neutralisée au préalable dans l'unité de réaction et on dose du formaldéhyde et de l'acide cyanhydrique en parallèle, le dosage de l'acide cyanhydrique étant plus lent que celui du formaldéhyde,
ou on transforme en continu l'α-alanine partiellement neutralisée avec du formaldéhyde et de l'acide cyanhydrique, la transformation étant réalisée dans une cascade de deux zones de réaction ou plus disposées les unes derrière les autres.

4. Procédé selon l'une quelconque des revendications 1 à 3, **caractérisé en ce qu'**on obtient la solution aqueuse d'α-alanine en partant d'α-alanine cristalline comme substance de départ et on la dissout ou la met en suspension dans de l'eau et la neutralise partiellement avec une base.

5. Procédé selon l'une quelconque des revendications 1 à 4, **caractérisé en ce qu'**on réalise la neutralisation partielle de l'α-alanine avec de l'hydroxyde de sodium ou de l'hydroxyde de potassium ou un mélange de lessive de soude caustique et de lessive potassique à un degré de neutralisation de 40 à 90% avec obtention d'une solution aqueuse concentrée, contenant 20 à 50% en poids d'alanine, par rapport au poids total de la solution aqueuse.

6. Procédé selon l'une quelconque des revendications 1 à 5, **caractérisé en ce qu'**on réalise le procédé sous forme d'un procédé semi-continu, **en ce que** l'α-alanine aqueuse partiellement neutralisée est disposée au préalable dans l'unité de réaction et du formaldéhyde et de l'acide cyanhydrique sont dosés en parallèle, le dosage de l'acide cyanhydrique étant plus lent que celui du formaldéhyde.

7. Procédé selon l'une quelconque des revendications 1 à 6, **caractérisé en ce que** la transformation de l'α-alanine partiellement neutralisée avec du formaldéhyde et de l'acide cyanhydrique est réalisée en continu dans une cascade de deux zones de réaction ou plus disposées les unes derrière les autres.

8. Procédé selon la revendication 7, **caractérisé en ce que** lesdites deux zones de réaction ou plus disposées les unes derrière les autres sont à chaque fois des réacteurs individuels.

9. Procédé selon la revendication 8, **caractérisé en ce que** lesdites deux zones de réaction ou plus disposées les unes derrière les autres sont à chaque fois des zones différentes au sein d'un seul réacteur.

10. Procédé selon la revendication 9, **caractérisé en ce que** l'α-alanine partiellement neutralisée est dosée dans la première zone de réaction, séparément ou sous forme prémélangée avec la quantité totale de formaldéhyde nécessaire pour la transformation uniquement ou, en plus, en parallèle, avec une quantité partielle de l'acide cyanhydrique nécessaire pour la transformation, et la quantité totale d'acide cyanhydrique ou le reste de la quantité d'acide cyanhydrique nécessaire pour la transformation est dosé(e) en parallèle ou sous forme prémélangée dans une zone de réaction consécutive dans le mélange réactionnel résultant de la première zone de réaction.

11. Procédé selon l'une quelconque des revendications 1 à 10, **caractérisé en ce qu'**on réalise la saponification de la solution aqueuse d'α-alanine-N,N-diacétonitrile obtenue par la synthèse de Strecker de manière telle qu'on la transforme d'abord avec de la lessive de soude caustique ou de la lessive potassique ou un mélange de lessive de soude caustique et de lessive potassique à 20 jusqu'à 80°C et ensuite à des températures ≥ 90°C avec libération d'ammoniaque en sels aqueux tri-métalliques alcalins de l'acide méthylglycine-N,N-diacétique.

12. Procédé selon l'une quelconque des revendications 1 à 11, **caractérisé en ce que** la solution aqueuse d'α-alanine est une solution aqueuse de L-α-alanine.
